# FASCICULE DE BREVET EUROPEEN

(11) **EP 1 043 952 B1**
(45) Date de publication et mention de la délivrance du brevet: **09.12.2009**
(21) Numéro de dépôt: 98962512.4
(22) Date de dépôt: 18.12.1998
(51) Int. Cl.: A61B 8/10

(54) **UTILISATION D'UN TRANSDUCTEUR ULTRASONORE POUR L'EXPLORATION ECHOGRAPHIQUE DU SEGMENT POSTERIEUR DU GLOBE OCULAIRE**
VERWENDUNG EINER UTRASCHALLSONDE ZUR AUSWERTUNG DER ECHOGRAPHIE DES HINTEREN SEGMENTES DES AUGAPFELS
USE OF AN ULTRASONIC TRANSDUCER FOR ECHOGRAPHIC EXPLORATION OF THE EYEBALL POSTERIOR SEGMENT

(30) Priorité: 18.12.1997 FR 9716071
(43) Date de publication de la demande: 18.10.2000
(73) Titulaire: Puech, Michel, 75007 Paris (FR)
(72) Inventeur: Puech, Michel, 75007 Paris (FR)
(74) Mandataire: Ahner, Francis
(86) Numéro de dépôt international: PCT/FR1998/002788
(87) Numéro de publication internationale: WO 1999/032036

(56) Documents cités:
- US-A- 5 551 432
- FOSTER F S ET AL: "ULTRASOUND BACKSCATTER MICROSCOPY OF THE EYE IN VIVO" PROCEEDINGS OF THE ULTRASONICS SYMPOSIUM, HONOLULU, DEC. 4 - 7, 1990, vol. VOL. 3, no. -, 4 décembre 1990, pages 1481-1484, XP000289858 MCAVOY B
- R.H.SILVERMAN ET AL: "Three-dimensional highfrequency ultrasonic parameter imaging of anterior segment pathology" OPHTHALMOLOGY, vol. 102, no. 5, mai 1995, pages 837-843, XP002074921 HAGERSTOWN, MD., US cité dans la demande
- C.J.PALVIN ET AL: "subsurface ultrasound microscopic imaging of the intact eye" OPHTHALMOLOGY, vol. 97, no. 2, février 1990, pages 244-250, XP002074922 HAGERSTOWN, MD., US cité dans la demande

## Description

La présente invention concerne l'utilisation d'un transducteur ultrasonore à fréquence élevée et à focalisation longue pour la réalisation d'un appareil et la mise en oeuvre d'un procédé d'exploration échographique du segment postérieur du globe oculaire, et plus particulièrement de la région maculaire.

C. Pavlin et F. Foster ont, les premiers, publié une série de travaux sur l'exploration oculaire par un appareil échographique de haute fréquence (50 MHz).

Depuis leurs premières publications sur un appareil de laboratoire en 1990 (C.J. PAVLIN, M.D. SHERAR , F.S. FOSTER : Subsurface ultrasound microscopic imaging of the intact eye. Ophthalmology 97 : 244,1990 et C. J. PAVLIN, M. EASTERBROOK, J.J. HURWITZ, K. HARASIEWICZ, F. STUART FOSTER : Ultrasound biomicroscopy in the assessment of anterior scleral disease American journal of Ophtalmology, November, 1993 ; 116 : 628-635), de nombreux travaux ont poursuivi cette innovation, avec apparition d'un appareil commercialisé par la firme Humphrey-Zeiss sous le nom d'U.B.M. (Ultrasound BioMicroscopy). De nombreux auteurs se sont alors emparés de la technique pour multiplier les publications sur l'exploration bidimensionnelle du segment antérieur in vivo.

Sous l'impulsion de J. Coleman, plusieurs articles de R. Silverman, N. Allemann et D. Reinstein ont dépassé le stade de la seule imagerie 2D pour lui associer une analyse du contenu fréquentiel.

La principale innovation de cette échographie de haute fréquence réside dans le fait que sa résolution axiale et latérale est d'environ 50 µm, avec la possibilité d'explorer les tuniques superficielles oculaires, in vivo, de façon comparable à une coupe histologique macroscopique.

La principale limitation de la technique est liée à la faible profondeur d'analyse des systèmes expérimentaux ou commerciaux. La plupart des publications sont faites avec des fenêtres de 4 mm de large sur 4 mm de profondeur, ce qui cantonne les observations au segment antérieur oculaire, sans atteindre la face postérieure du cristallin, ou bien, à la partie toute antérieure du segment postérieur, en décalant le transducteur sur la pars plana. En effet, plus la fréquence du faisceau ultrasonore augmente, plus son atténuation, par les milieux traversés, se majore.

S'il apparaît donc irréaliste d'espérer obtenir une résolution de 50 microns au niveau rétinien, avec des transducteurs de 50 à 80 MHz , notre souci a été l'amélioration sensible de la résolution des images du segment postérieur. En effet, en comparant l'oeil à un appareil photographique, si le segment antérieur joue le rôle de l'objectif avec son lot de problèmes optiques, le segment postérieur détermine les potentialités visuelles, grâce au tissu essentiel que représente la rétine jouant le rôle de pellicule photographique.

La nécessité d'améliorer la qualité de l'exploration rétinienne est liée à deux notions d'importance :
- il n'existe pas, à l'heure actuelle, de possibilité de réaliser des greffes de rétine, malgré des travaux de recherche encourageants, alors que le remplacement des éléments essentiels du segment antérieur est possible (greffe de cornée, opération de la cataracte),
- la plus forte concentration de cellules visuelles rétiniennes se situe sur une très faible surface appelée macula. De son intégrité dépendent les capacités visuelles.

Son exploration est, le plus souvent pratiquée, par des moyens optiques, comme la biomicroscopie et l'angiographie. Ces explorations sont cependant limitées par les problèmes de transparence des milieux oculaires (cataracte, hémorragies) et ne fournissent qu'une imagerie plane de la rétine

L'échographie en mode B donne une image en coupe de la rétine, et explore, de façon plus précise, les relations vitréo-rétiniennes. Cependant, si la résolution des appareils de 10 MHz est suffisante pour explorer la rétine dans son ensemble, elle ne permet pas une analyse de la région maculaire aussi fine que l'angiographie.

En outre, il est possible de réaliser un examen du segment antérieur par échographie à 10 MHz, il faut pour cela réaliser une immersion avec des cupules appropriées, afin de ramener, sur le segment antérieur, la zone focale située à 23 mm.

Cependant, depuis l'apparition des appareils de haute fréquence, la différence de résolution des images est telle, que l'exploration du segment antérieur par échographie 10 MHz est reléguée au second plan.

Le seul appareil commercialisé au niveau mondial (UBM) est un appareil de 50 MHz qui fournit des images 2D sur une profondeur maximale de 4mm avec une résolution axiale de 50 microns. De plus, cet échographe affiche une fenêtre de visualisation limitée à 4 mm de large, insuffisante pour explorer le segment antérieur en entier, sur une seule coupe de B-scan. La possibilité de produire des images 3D, et de réaliser une caractérisation tissulaire, n'a pas été prévue par le fabriquant.

Les premiers travaux de C. Pavlin et S. Foster en 1990, ont été réalisés avec un appareil de laboratoire utilisant des sondes de 50 à 100 MHz afin de produire une imagerie 2D du segment antérieur. Grâce à cet appareil, différents travaux d'exploration ont été publiés concernant l'anatomie et la physiologie du segment antérieur (C.J. PAVLIN, J.A. McWHAE, J.A. McGOWAN, F.S. FOSTER. Ultrasound biomicroscopy of anterior segment tumors. Ophthalmology 99: 1222,1992 et C. TELLO, T. CHI, G. SHEPPS, J. LIEBMANN, R. RITCH : Ultrasound biomicroscopy in pseudophakic malignant glaucoma. Ophtalmology, September 1993 ; volume 100, 9 : 1330-1334.), avec notamment l'étude de l'angle irido-coméen et de ses variations morphologiques, dans les pathologies glaucomateuses. Plusieurs publications ont imagé les tumeurs de l'iris, avec des coupes en B-scan d'une résolution de 50 à 60 microns, permettant de visualiser l'échostructure de ces lésions, ainsi que de mieux en définir leurs limites (C.J. PAVLIN, J.A. McWHAE, J.A. McGOWAN, F.S. FOSTER. : Ultrasound biomicroscopy of anterior segment tumors.Ophthalmology 99 :1222, 1992 et L. ZOGRAFOS, L. CHAMOT, L. BERCHER : Contribution of ultrasound biomicroscopy to conservative treatment of anterior uveal melanoma. Klin. Monast. Augen. 1996; 208(5) :414-7).

Les mêmes auteurs ont montré que l'accès au segment antérieur avec une haute résolution permettait la surveillance des patients après chirurgie de la cataracte avec par exemple, la surveillance de la position des implants intra-oculaires. De même, après les interventions pour glaucome, le contrôle de la fonctionnalité du geste chirurgical a représenté un grand progrès.

La version commerciale de cet appareil (UBM de Zeiss-Humphrey) a servi de base à de très nombreuses publications internationales, comme celles de C. Tello sur la physiologie du glaucome pigmentaire et du glaucome malin(C. TELLO, T. CHI, G. SHEPPS, J. LIEBMANN, R. RITCH : Ultrasound biomicroscopy in pseudophakic malignant glaucoma. Ophtalmology, September 1993 ; volume 100, 9 : 1330-1334.).

L'équipe de J. Coleman a utilisé un appareil de recherche avec un transducteur de 60 MHz à partir duquel, R. Silverman et D. Reinstein ont publié en 1992 et 1993 (D. Z. REINSTEIN, R. H. SILVERMAN, S. L. TROKEL,D. J. COLEMAN : Cornealpachymetric topography. Ophtalmology, March 1994 ; volume 101, 3 : 432-438. et D. Z. REINSTEIN, R. H. SILVERMAN, M. J. RONDEAU, D. J. COLEMAN : Epithelial and corneal measurements by high-frequency ultrasound digital signal processing. Ophtalmology, January 1994 ; volume 101, 1 : 140-146.) plusieurs travaux sur l'amélioration de l'exploration cornéenne avec utilisation de traitements du signal, permettant, par exemple, de réaliser des cartographies cornéennes représentant les épaisseurs des différentes couches cornéennes avec une résolution de 2 microns.

Cette même équipe ayant déjà été une des plus avancée en matière de caractérisation tissulaire des tumeur oculaires par échographie 10MHz dans les années 70 et 80 (D.J. COLEMAN, F.L. LIZZI. : Computerised ultrasonic tissue characterisation of ocular tumors. AM.J. Ophthalmol.1983 ;96 :165-75), il était naturel que, par la voie de N. Allemann, les premières publications sur l'analyse quantitative du segment antérieur à haute fréquence soient issus de ce même laboratoire en 1993 (N. ALLEMAN, W. CHAMON, R. H. SILVERMAN, D. T. AZAR, D.Z. REINSTEIN, W.J. STARK, D.J. COLEMAN : High-frequency ultrasound quantitative analyses of corneal scarring following excimer laser keratectomy. Arch Ophtalmol 1993 ; 111 : 968-973 et N. ALLEMAN, R. H. SILVERMAN, D. Z. REINSTEIN, D.J. COLEMAN : High-frequency ultrasound imaging and spectal analysis in traumatic hyphema. Ophtalmology, September 1993 ; volume 100, 9 : 1351-1357).

En 1995, R. Silverman publiait une reconstitution 3D des images paramétriques de rétrodiffusion réalisées à 50 MHz de différentes pathologies du segment antérieur (R.H. SILVERMAN ET AL. : Three dimensional high frequency ultrasonic parameter imaging of anterior segment pathology. Ohthalmology, 1995, 102, 837-843).

En ce qui concerne l'analyse du segment postérieur par échographie de haute fréquence, la revue de la littérature ne retrouve que quatre publications, toutes réalisées avec un UBM sur la partie très antérieure du segment postérieur :
- T. Boker (T. BOKER, M. SPITZNAS. : Ultrasound biomicroscopy for examination of the sclerotomy site after pars plana vitrectomy. Am. J. Ophthalmol. 1994 :15 ; 118(6) :813-5) en 1994 publie une étude du site de sclérotomie après vitrectomie par la pars plana.
- En 1995, C. Azzolini (C. AZZOLINI. L. PIERRO, M. CODENOTTI, F. BANDELLO , R. BRANCATO. : Ultrasound biomicroscopy following the intraocular use of silicone oil. International Ophthalmology 1995-96, 19(3) :191-5) a imagé la présence de résidus de silicone intra -vitréen dans la partie antérieure de la cavité vitréenne.
- En 1996, L. Zografos (L. ZOGRAFOS, L. CHAMOT, L. BERCHER : Contribution of ultrasound biomicroscopy to conservative treatment of anterior uveal melanoma. Klin. Monast. Augen. 1996; 208(5) :414-7) a publié une étude par UBM de 55 cas de mélanomes de l'uvée situés au contact ou à proximité du corps ciliaire. La conclusion de ce travail fait apparaître que la forte atténuation du signal ultrasonore de haute fréquence limite l'utilisation de l'UBM aux structures situées dans le voisinage direct de la paroi oculaire. Cependant la contribution de l'échographie de haute fréquence, dans le suivi des mélanomes de l'uvée après traitement conservateur, est apparue comme considérable.
- A. Minamoto, en 1997, (A. MINAMOTO, K.E. NAKANO , S. TANIMOTO. : Ultrasound biomicroscopy in the diagnosis of persistent hypotony after vitrectomy. Am. J. Ophthalmol. 1997 ;123(5)711-3) étudie le décollement du corps ciliaire, situé à la jonction entre segment antérieur et segment postérieur, en cas d'hypotonie après vitrectomie.

De nombreuses publications ont pour sujet l'étude du segment postérieur en échographie 10 MHz, avec notamment l'exploration de la région maculaire.

La revue récente de la littérature internationale ne retrouve aucune publication sur l'exploration de la région maculaire par échographie de fréquence plus élevée que les sondes de 10 MHz couramment utilisés.

L'originalité de notre travail réside dans l'amélioration de la résolution d'analyse de la macula, qui permet d'améliorer l'iconographie 2D in vitro avec possibilité de produire des images 3D.

Nous divulguons l'utilisation d'un transducteur ultrasonore de fréquence nominale d'excitation supérieure à 20 MHz, de préférence comprise entre 50 et 80 MHz, à focalisation longue supérieure à 10 mm, de préférence de l'ordre de 25 mm, pour la réalisation d'un appareil d'exploration échographique de tissus ou organes du corps humain ou animal, notamment du globe oculaire, en particulier du segment postérieur du globe oculaire et plus particulièrement de la région maculaire, ainsi que des tissus situés en arrière du globe oculaire comme les muscles oculomoteurs, la graisse orbitaire et le nerf optique.

De façon tout à fait surprenante, on a constaté que l'analyse en échographie haute fréquence du segment postérieur du globe oculaire, considérée comme impossible avec les appareils existants, à cause de leur limite de pénétration tissulaire de 4 à 5 millimètres de profondeur était en fait réalisable grâce à l'utilisation d'une sonde de 50 MHz focalisée à environ 25 mm. L'analyse du segment postérieur du globe oculaire et, en particulier, de la région maculaire a donc été rendue possible, dans d'excellentes conditions, grâce à la présente invention.

C'est pourquoi la présente invention concerne l'utilisation d'un transducteur ultrasonore de fréquence nominale d'excitation supérieure à 20 MHz, de préférence comprise entre 50 et 80 MHz, à focalisation de l'ordre de 20 à 25 mm, pour la réalisation d'un appareil d'exploration échographique de tissus ou organes du corps humain ou animal, notamment du globe oculaire, en particulier du segment postérieur du globe oculaire et plus particulièrement de la région maculaire, ainsi que des tissus situés en arrière du globe oculaire comme les muscles oculomoteurs, la graisse orbitaire et le nerf optique.

A titre d'exemple de tissus ou d'organes susceptibles d'être également explorés dans le cadre de la présente invention, on peut citer les différentes couches de la peau, les muscles et les tendons, la thyroïde, le foie et le pancréas.

Un mode de réalisation de l'invention concerne également l'utilisation d'un transducteur ultrasonore, décalé sur la pars plana pour éviter l'absorption du faisceau ultrasonore par le cristallin de l'oeil, lorsque l'on souhaite explorer le segment postérieur du globe oculaire ainsi que les tissus situés en arrière dudit globe oculaire.

De façon préférentielle, le transducteur ultrasonore est décalé sur le pars plana pour éviter l'absorption du faisceau ultrasonore par le cristallin de l'oeil.

Enfin, nous divulguons également un appareillage d'exploration échographique comportant un système émetteur-récepteur haute fréquence (20 à 200 MHz) couplé à un transducteur ultrasonore à focalisation longue supérieure à 10 mm de préférence de l'ordre de 20 mm, et un système d'amplification et de mémorisation du signal radiofréquence restituée après l'exploration, de préférence associé à un système enregistreur du signal amplifié et/ou à un système de traitement du signal sous forme d'images, et/ou à un système de traitement du signal en vue de réaliser une caractérisation tissulaire.

La présente invention s'étend également à un appareillage d'exploration échographique comportant un système émetteur-récepteur haute fréquence (20 à 200 MHz) couplé à un transducteur ultrasonore, la focalisation dudit transducteur ultrasonore étant de l'ordre de 20 mm à 25 mm, et un système d'amplification et de mémorisation du signal radiofréquence restituée après l'exploration, de préférence associé à un système enregistreur du signal amplifié et/ou à un système de traitement du signal sous forme d'images, et/ou à un système de traitement du signal en vue de réaliser une caractérisation tissulaire.

Selon une caractéristique avantageuse de la présente invention, un tel appareil comprend un transducteur ultrasonore agencé sous la forme d'une sonde pilotée de manière à se déplacer à proximité de la paroi antérieure oculaire. Ce déplacement peut être réalisé selon deux axes orthogonaux, ou encore selon un déplacement arciforme.

Cette sonde peut être focalisée selon un troisième axe orthogonal aux deux axes orthogonaux de déplacement, ou être focalisée sans déplacement à l'aide d'un système de focalisation électronique.

Avantageusement selon l'invention, la sonde peut être protégée par une membrane en matière plastique.

On décrira, ci-après, les matériels et les méthodes expérimentales utilisées, notamment en référence aux dessins annexés illustrant les principaux résultats observés.

L'expérimentation a été conduite sur différents types de globes oculaires.

### Les globes oculaires d'animaux :

Les premiers tests ont étés réalisés sur des globes de lapins de Nouvelle-Zélande prélevés immédiatement après leur euthanasie par surdose de pentobarbital. Avant leur analyse échographique, ils ont été placés dans une solution de Chlorure de Sodium à 9/1000 et positionnés sous le capteur, par un dispositif d'immobilisation permettant d'observer les mêmes régions d'intérêt avec différents capteurs. Ces globes ont servis à la comparaison des capteurs avec une focalisation sur la cornée et le segment antérieur.

Des yeux de porc fixés dans une solution de Bouin ont été utilisés pour les premiers essais de la sonde de 50 MHz focalisée à 25mm. En effet, ces globes oculaires ont une taille très voisine des globes oculaires humains et représentent un bon modèle pour l'exploration du segment postérieur.

Les globes oculaires humains :
Nous avons utilisé 6 globes oculaires humains prélevés sur trois personnes différentes décédées après avoir légué leurs corps à la Science :
   - Les globes 1 et 2 provenaient d'un homme de 75 ans décédé 2 mois avant l'énucléation sans pathologie particulière.
   - Les globes 3 et 4 provenaient d'une femme de 48 ans décédée par accident vasculaire cérébral, 14 jours avant l'énucléation.
   - Les globes 5 et 6 provenaient d'un homme de 41 ans décédé par accident de la voie publique, 7 jours avant l'énucléation.

La conservation de ces corps a utilisé la congélation, ce qui a entraîné des artefacts oculaires notables sur la cornée, sous forme de stries cornéennes blanchâtres, ainsi que sur le vitré, sous forme de fibres intra-vitréennes.

L'énucléation a été réalisée par dissection conjonctivale au limbe, crochetage et section des muscles oculomoteurs sans exercer de traction sur le globe lui même, puis section du nerf optique et des artères et nerfs ciliaires postérieurs au ciseau courbe à bout mousse.
Pour chaque paire d'yeux, l'un des globes a été immédiatement placé dans du sérum physiologique pour être étudiés frais, dans l'heure qui a suivi l'énucléation. En fin d'étude échographique, l'oeil frais a été fixé par une solution de Bouin, au plus tard le soir même de l'énucléation.

Le second globe a été immédiatement fixé dans une solution de Bouin au moment du prélèvement afin d'être analysé de façon différée.

L'aspect des 6 globes était macroscopiquement comparable, avec une importante hypotonie, nécessitant un remplissage oculaire au sérum par seringue à insuline pour leur redonner une forme sphérique. Nous avons réalisé une injection de quelques centimètres cubes dans la cavité vitréenne, par une injection en pars plana pour éviter de provoquer un décollement de rétine hyatrogène. Puis nous avons complété le remplissage oculaire par une injection de sérum en chambre antérieure, réalisée en incision cornéenne inverse autoétanche. Cette dernière injection a pour but d'éviter un déplacement du cristallin vers l'avant sous l'effet d'une seule injection intra-vitréenne, avec comme conséquence la fermeture artificielle de l'angle irido-cornéen et l'aplatissement de la chambre antérieure.

Chaque globe a été analysé par échographie classique (10 MHz) puis, avec le microscope ultrasonore (50 MHz). Après leur fixation, les globes oculaires humains ont été adressés au laboratoire pour analyse histologique.

On décrira, ci-après, brièvement les différents appareils utilisés.

### Le microscope ultrasonore

Les différents transducteurs de PVDF (Copolymère de fluorure de vinylidène) sont excités par un émetteur/récepteur (Panametrics 5900) pour générer un faisceau ultrasonore de large bande.

Le signal ultrasonore rétrodiffusé est amplifié puis digitalisé à une fréquence d'échantillonage de 400 MHz sur 8 bits, par un oscilloscope Lecroy 9450A .Pour chaque acquisition, le signal est moyenné par l'oscilloscope afin d'améliorer le rapport signal/bruit. Ensuite un ordinateur PC Dell 486 traite les A-scan et reconstruit les images en mode B, après calcul de l'enveloppe du signal radio fréquence par transformée de Hilbert.

Des moteurs (Microcontrôle Newport) à pas de haute précision (0.1 µm), se déplaçant dans deux axes de translation perpendiculaires X et Y, permettent d'acquérir des informations tridimensionnelles. Ces moteurs sont pilotés par le PC.

Pour les acquisitions en trois dimensions du segment postérieur, le pas de déplacement en X a été choisi tous les 100 µm avec un pas de 200 µm sur l'axe des Y.

Le champ d'acquisition était en moyenne de 14 mm (sur l'axe des X), sur 8 mm (sur l'axe de Y). Avec 140 lignes sur l'axe des X et 40 lignes sur l'axe des Y. Chaque ligne comportait 2000 ou 4000 points en fonction de la fréquence d'échantillonnage ( 200 ou 400 MHz ).

La profondeur d'acquisition était en moyenne de 8mm.

### Les transducteurs en PVDF :

Pour l'exploration spécifique du segment antérieur nous avons utilisé des sondes de distance focale assez courte :
- Sonde Krautkramer de 80 MHz d'émission avec une distance focale de 7,5 mm un diamètre de 3 mm et une fréquence centrale à la focale de 60MHz. Sa résolution axiale est de 20 à 30 µm, avec une résolution latérale de 60 à 80 µm.
- Sonde Sofratest de 33 MHz d'émission avec une distance focale de 12.5 mm, un diamètre de 6 mm et une fréquence centrale à la focale de 26 MHz. Sa résolution axiale est de 64 µm, avec une résolution latérale de 120 µm.

Pour l'exploration rétinienne nous avons utilisé une sonde qui possède une distance focale suffisante pour que le faisceau ultrasonore puisse traverser le globe oculaire par la pars plana. Il s'agit d'une sonde Panametrics de 50 MHz d'émission avec une distance focale de 25 mm., un diamètre de 6 mm et une fréquence centrale à la focale de 28 MHz. Sa résolution axiale est de 70 µm pour 125 µm de résolution latérale, à la focale, dans le sérum physiologique

Avantageusement selon l'invention, toutes ces sondes ont été isolées du bain d'immersion par un film ou membrane en matière plastique (du type film alimentaire), recouvrant le transducteur afin d'éviter l'altération de la partie active de ce transducteur par contact avec la solution de couplage entre le globe oculaire et la sonde échographique.

Actuellement, dans les appareils de type connu, les sondes utilisées sont plongées directement dans le bain d'immersion sans protection particulière du transducteur. Ceci nécessite obligatoirement la stérilisation ultérieure du transducteur avant toute nouvelle utilisation. Une telle stérilisation n'est pas réalisée de manière complètement efficace.

### L'appareil échographique 10 MHz

Les images rétiniennes obtenues par le banc de haute fréquence sont comparées aux images obtenues en échographie classique 10 MHz dont la sonde est focalisée à 23 mm, avec une résolution axiale et latérale d'environ 1 mm. Cette exploration est la seule disponible en pratique courante.

Nous avons iconographié les globes prélevés avec un appareil Compact (Société B.V.I.) juste avant l'acquisition des données par le microscope ultrasonore. Cet appareil fait partie des échographes oculaires les plus performants à l'heure actuelle.

L'analyse de la réponse en fréquence des signaux provenant de la paroi postérieure :
Nous avons analysé les images rétiniennes obtenues par le biomicroscope ultrasonore, pour connaître la réponse en fréquence de la paroi postérieure. Afin d'analyser de façon spécifique la réponse en fréquence de la paroi oculaire postérieure, il nous a fallu sélectionner une zone d'intérêt.

En revenant au signal radio fréquence, nous avons sélectionné, pour chaque globe, dix images prises au hasard. Les différents A-scan de ces images ont été seuillés, afin de détecter le premier pic pariétal. A partir de ce premier pic, la ligne de A-scan à été translatée vers le zéro, avec report de la partie vitréenne en fin de ligne, où l'on a attribué une valeur nulle à tous les points. Ce seuillage avec réduction, à permis de passer d'une image pariétale concave vers l'avant en mode B, à une image pariétale verticale, débutant à zéro. Cette étape permet une meilleure sélection de la fenêtre d'analyse, focalisée sur la partie antérieure de la paroi maculaire.

Une transformée de Fourier rapide (FFT) nous a permis de passer dans le domaine fréquentiel pour analyser ligne par ligne la réponse fréquentielle.

Pour s'assurer que les point analysés correspondaient bien à des points pariétaux nous avons fenêtré l'analyse à partir du point 100 jusqu'au point 128 pour analyser plutôt la partie antérieure de la paroi postérieure où se situe la rétine. C'est ainsi que nous avons obtenu la courbe de réponse en fréquence, ligne par ligne, pour la fenêtre considérée.

Pour chaque coupe, toutes les lignes ont été moyennées. Puis nous avons fait la moyenne sur 10 coupes, prises au hasard sur chaque oeil.

### Imagerie rétinienne 3D

L'acquisition des images rétiniennes a été réalisée grâce au microscope ultrasonore avec un balayage linéaire suivant les axe X et Y. Les données obtenues ont été traitées de deux façons pour améliorer la restitution des images, avec le souci d'une présentation facilement reconnaissable par le clinicien.

La première présentation des données a utilisé une représentation en C-scan qui permet de donner une image plane du fond d'oeil exploré par échographie. Nous avons étudié, de façon comparative, les différentes possibilités d'aboutir à une image maculaire compréhensible par les ophtalmologistes qui sont habitués à des représentations rétiniennes de type angiographique. Le fond d'oeil y est retranscrit sous la forme d'une photographie à plat, alors que la rétine suit une concavité à ouverture antérieure.

Le C-scan permet, à partir d'une acquisition échographique 3D, d'extraire les plans transverses aux axes X et Y. En sélectionnant une fenêtre de plusieurs de ces plans parallèles, le C-scan va réduire l'information en une seule image plane, après avoir utilisé un opérateur de type maximum, minimum, moyenne ou médiane sur l'amplitude du signal.

La deuxième représentation des données a utilisé une station Silicon Graphics travaillant sous Unix, avec le logiciel AVS. La séquence de traitement suivante :
- Lecture des données
- Moyennage sur 49 points puis réduction des données en conservant 1 valeur sur 7, de façon à réduire la taille des images qui étaient sur-échantillonées.
- Conversion des valeurs de l'échelle -32000 à + 32000 en échelle de 0 à 255 niveau de gris.
- Seuillage : avec un seuil de 15 appliqué sur les valeurs de 0 à 255 pour faire ressortir les zones d'intérêt.
- Opération d'ouverture morphologique, avec un élément structurant horizontal de 31 pixels de long, afin de supprimer les images vitréennes liées à des brides intra-vitréennes venant au contact de la rétine.
- Opération d'ouverture morphologique avec un élément structurant vertical de 3 pixels de long, pour supprimer les artefacts liés à des attaches vitréo-rétiniennes.
- Remplissage des trous de taille inférieure à 200 pour les objets noirs. (Basé sur l'étiquetage des pixels par régions connexes puis suppression des petits îlots).
- Elimination des objets de taille inférieure à 500 pixels pour les objets blancs. Ces deux dernières opérations ont pour but de supprimer les petits îlots de pixels noirs ou blancs, de façon à ne retenir que le contour rétinien qui représente la séparation entre la cavité vitréenne et la paroi oculaire postérieure.
- Opération de fermeture mathématique en croix pour réduire l'irrégularité des contours.
- Visualisation 3D de la surface des objets ainsi obtenus. La représentation des images a été restituée par une imprimante laser couleur.

### Histologie

Les 6 globes humains on été adressés au laboratoire d'anatomocytopathologie oculaire de l'Hôtel Dieu de Paris.

Tous les yeux ont été fixés dans le Bouin, la moitié immédiatement après le prélèvement, l'autre moitié après analyse échographique sur globe frais sans dépasser 12 heures entre le prélèvement et la fixation.

Les globes ont été techniqués par découpe et inclusion dans la paraffine puis des coupes semi-fines (2 µm d'épaisseur) ont été réalisées avec un ultramicrotome (Reichert OMU).

Après coloration des différentes lames au HES l'analyse a été conduite au microscope optique.

Lors de l'exploration du segment postérieur, on a observé les résultats suivants.

La possibilité d'utiliser une sonde de 50 MHz focalisée à 25 mm (28 MHz à la focale) nous a permis de positionner le transducteur sur la pars plana des différents globes animaux puis humains de façon à jeter les bases d'une exploration maculaire in vivo, non invasive, utilisable en pratique courante.

Le faisceau ultrasonore traverse, dans un premier temps, la paroi oculaire périphérique, puis toute la cavité vitréenne pour arriver enfin au niveau de la paroi oculaire postérieure. En positionnant le globe de façon à exposer la pars plana nasale en regard du transducteur, l'exploration postérieure se fait directement sur la zone maculaire située en temporal de la papille. Cette papille est un repère identifiable en échographie 10 MHz comme à plus haute fréquence.

Les premiers essais réalisés sur globe de porc fixé ont permis de visualiser la paroi postérieure, malgré la mauvaise qualité du tissus rétinien après fixation. Cependant, les tests réalisés nous ont aidé à régler les différents paramètres de la chaîne d'acquisition des données échographiques, pour optimiser les résultats de l'exploration sur globe humain.

Les meilleures images ont été obtenues avec la sonde Panametrics de 50 MHz focalisée à 25 mm, connectée au générateur dont le damping était réglé à 50 ohms, la puissance à 2 microjoules. L'oscilloscope Lecroy réalisait un moyennage sur 30 mesures par point.

Les moteurs se déplaçaient tous les 100 microns sur l'axe des X et tous les 200 microns sur l'axe des Y lorsqu'une acquisition 3D était lancée.

La longueur axiale des globes oculaires humains étant en moyenne de 23,5 mm, le positionnement du transducteur à l'ora serrata permet d'amener la focale de la sonde sur la rétine maculaire. L'analyse de coupes réalisées sur les globes 1 et 2 comparées aux coupes maculaires obtenues par échographie 10 MHz montre une meilleure résolution des images dues à la sonde Panametrics de 50 MHz. :

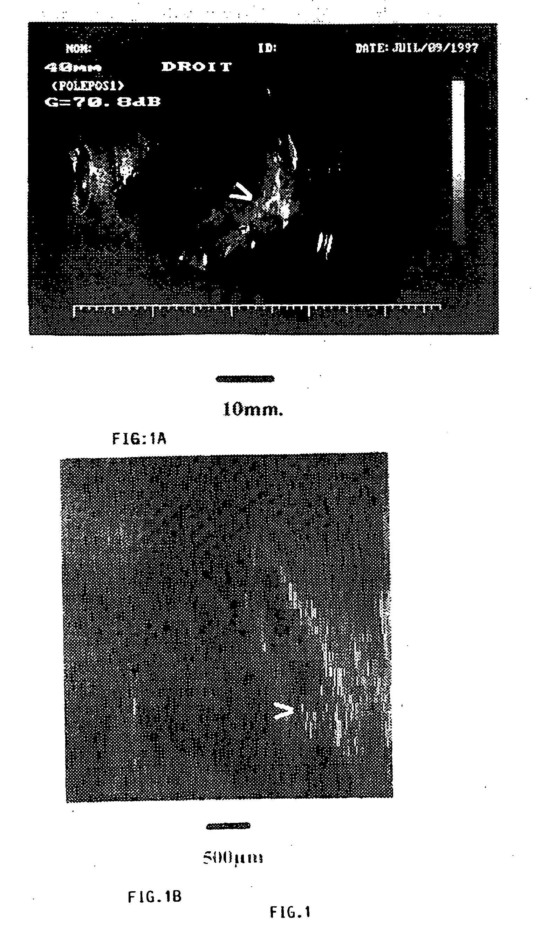

La Figure 1 montre la comparaison entre l'image d'une coupe 8-scan classique 10 MHz (cliché du haut) où l'on peut noter sur le globe N° 1 la présence d'un remaniement vitréen avec légères ondulations de la paroi rétinienne.

L'image maculaire de plus haute fréquence (cliché du bas), montre une des premières images que nous avons obtenues avec un pli rétinien que l'on devine en inférieur de la coupe. Sur ce pli rétinien s'attache une fine bride de vitré.

Les images obtenues sur les globes 3, 4, 5 et 6 sont encore plus démonstratives :

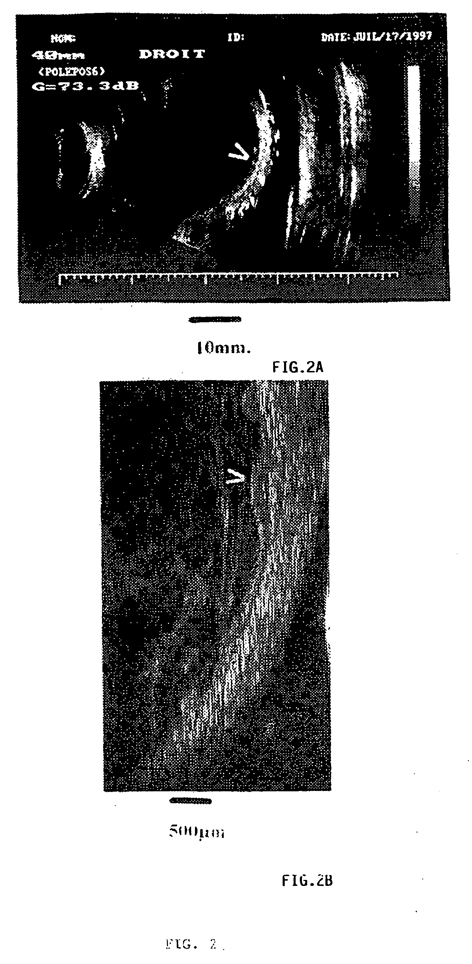

La Figure 2 montré une coupe échographique de 10 MHz passant par la région maculaire du globe N° 6 avec petite ondulation de la paroi postérieure.

La coupe de haute fréquence passant par la même zone, montre de façon plus précise la déformation de la paroi postérieure avec présence de tractions vitréennes insoupçonnées par l'échographie classique.

Il est à noter que ces images échographiques ont été obtenues avec une sonde de haute fréquence recouverte par un film ou une membrane en matière plastique, alors que la sonde de l'appareil actuellement commercialisé pour l'exploration du segment antérieur utilise des transducteurs non protégés vis-à-vis du bain d'immersion.

### Analyse de la réponse en fréquence des signaux rétiniens

En connaissant la fréquence d'émission de la sonde Panametrics (50 MHz), ainsi que sa fréquence maximale à la focale dans le sérum (28 MHz), nous avons voulu mesurer la fréquence maximale véritablement retrouvée au niveau de la focale après traversée des milieux oculaires. En effet, de cette fréquence dépend la résolution spatiale des images du segment postérieur. Après seuillage à 100 des lignes radio fréquence et report à zéro du premier point rétinien, la FFT nous a permis de retrouver une courbe moyenne des réponses en fréquence de chaque image rétinienne.

En réalisant cette opération sur dix coupes prises au hasard pour chacun des 6 globes oculaires, on obtient la valeur moyenne de 21 MHz qui est légèrement inférieure aux 28 MHz de cette même sonde, mesurés à la focale dans du sérum. Cette modification en fréquence du signal est due à la traversée des structures oculaires par l'onde ultrasonore (paroi limbique et cavité vitréenne. dont la structure collagénique atténuante a été dégradée par les remaniements post mortem liés à l'autolyse des tissus et à la congélation des corps).

Les globes 3 et 4 ont une réponse en fréquence plus élevée (22,033 et 21,638 MHz ) : peut être expliquée par un meilleur état vitréen qui produirait une atténuation moins marquée du faisceau ultrasonore.

Ce résultat prouve que le microscope ultrasonore utilisé pour cette étude, permet d'obtenir, au niveau de la paroi postérieure des globes humains analysés, une fréquence de 21 MHz, au moins égale à deux fois la fréquence couramment retrouvée avec les sondes classiques de 10 MHz dont l'atténuation en fréquence du signal est beaucoup plus réduite.

Les 21 MHz obtenus à la focale avec notre appareil, nous permettent d'obtenir une résolution au moins deux fois meilleure pour l'exploration de la paroi oculaire postérieure, que la résolution de l'appareil 10 MHz classique.

L'acquisition 3D a permis d'appliquer sur les différents globes oculaires humains la représentation par C-scan qui donne une vue à plat du fond d'oeil.

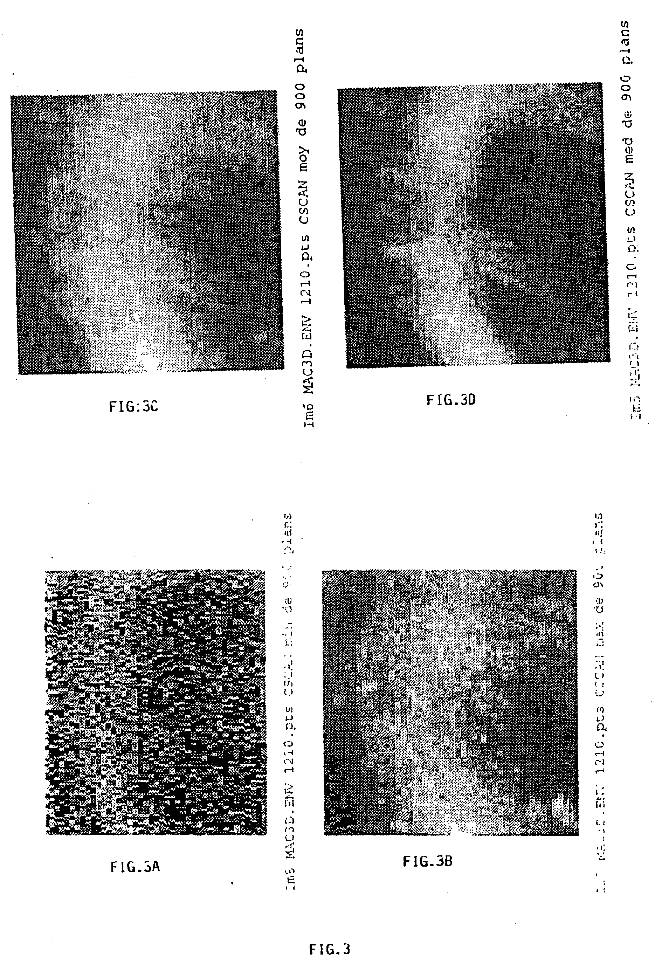

La Figure 3 montre les différents C-scan obtenus en faisant varier le paramètre d'amplitude sélectionné dans la fenêtre. C'est la valeur médiane appliquée à l'amplitude du signal qui donne la meilleure information. Il s'agit de l'exemple du globe N° 3, pour lequel, nous avons sélectionné une fenêtre de 900 plans qui correspond à la partie antérieure de la paroi oculaire postérieure avec, à sa surface, la couche rétinienne.
- L'image du haut montre l'application d'un opérateur de type minimum aboutissant à une image difficilement interprétable.
- La deuxième image correspond à l'application d'un opérateur de type maximum sur les mêmes 900 plans avec apparition d'une image floue
- La troisième image est le résultat de l'opérateur de type moyenne qui permet d'identifier l'aspect du fond d'oeil ressemblant à l'observation que les ophtalmologistes font en pratique courante avec les différentes loupes d'examen. Il est possible de reconnaître en haut de l'image une structure arrondie hypo-échogène qui correspond à la papille optique. Partant de la papille, il est possible de reconnaître une «coulée» plus échogène se dirigeant, de façon oblique en bas et à gauche ; cette image correspond au pli rétinien le plus important retrouvé sur les différents B-scan passant par cette zone.
- La dernière image est la meilleure traduction que nous ayons obtenue, pour rendre compte, de façon didactique, de l'état rétinien maculaire. Cette image a été obtenue en appliquant un opérateur de type médiane sur les mêmes 900 plans de cette acquisition. Le pli rétinien et la papille apparaissent presque réels et très comparables aux images cliniques et angiographiques classiques.

Il est possible, grâce au C-scan, de sélectionner une fenêtre de plans situés à des profondeurs différentes pour aller, par exemple, étudier les plans sous rétiniens ou les couches papillaires profondes. Cet avantage permettra à ce système d'être très complémentaire de l'exploration angiographique en donnant des informations sur les plans profonds de la macula, quelque soient les conditions de transparence des milieux. En effet, l'interprétation des clichés angiographiques est vite limitée en cas, par exemple d'hémorragie du vitré ou d'hémorragie dans les couches rétiniennes superficielles.

Une des applications les plus intéressantes pourra être l'exploration des membranes néovasculaires sous maculaires en cas de Dégénérescence Maculaire Liée à l'Age. Cette pathologie de plus en plus fréquente et très invalidante fait l'objet de traitements chirurgicaux nouveaux, et très prometteurs, recourant à l'extraction de ces membranes par dissection microscopique sous rétinienne. L'exploration angiographique, couplée à une imagerie échographique de niveau histologique, donnera au chirurgien une meilleure connaissance de la forme, de l'étendue et de l'épaisseur de ces membranes, tout en précisant leurs rapports sous rétiniens. L'apport de ce type d'imagerie permettra aussi de mieux en apprécier la physiopathologie.

Le globe Humain N° 3 a été choisi pour servir de base à la reconstitution 3D ; en effet, ce globe est celui qui présente un pli rétinien le plus facilement identifiable.

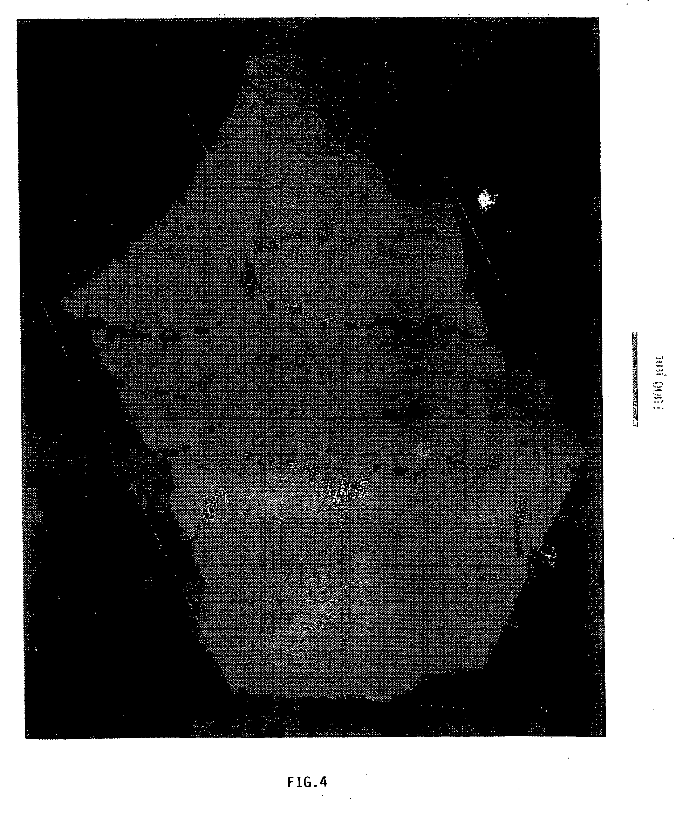

La Figure 4 montre le résultat de l'impression d'une des vues 3D obtenue en fin d'application du programme AVS. Il est possible de bien identifier le pli rétinien formant un relief très net, en avant du plan rétinien. Cependant, ses contours sont irréguliers et majorés probablement par l'imperfection de la segmentation entre rétine et vitré.

L'intérêt d'une telle imagerie 3D est de permettre une vision plus globale des pathologies du segment postérieur, surtout lorsqu'elles entraînent un épaississement, dont il est facile de mesurer le volume. Les applications cliniques seront rapidement tournées vers les Dégénérescences Maculaires Liées à l'Age qui sont très fréquentes, mais aussi vers les tumeurs du segment postérieur. Pour ces dernières, l'avantage de notre système est de conserver toutes les informations du signal radio fréquence, afin de réaliser une étude, non seulement sur le volume et sur les extensions de ces lésions, mais aussi d'en réaliser une caractérisation tissulaire par analyse des paramètres ultrasonores d'atténuation et de diffusion.

L'analyse histologique des différents globes oculaires humains a montré de nombreuses altérations autolytiques visibles macroscopiquement sur tous les globes, avec nécrose étendue de la rétine ayant entraîné une perte de substance de la rétine antérieure, mais celle-ci reste visible au pôle postérieur où, elle semble épaissie, avec des plicatures, et des zones de décollement par artefacts. Elle est faite de couches nucléaires, entre couches très autolysées. Cet empilement en couches permet cependant de la reconnaître sans difficulté. Les zones plissées de la rétine et d'épaississement focalisé de la rétine sont visibles de part et d'autre de la rétine juxta-papillaire, la seule qui ait résistée à l'autolyse et à l'ouverture oculaire.

Le plan choroïdien est bien visible, faisant suite au plan ciliaire, la choroïde ayant une structure relativement préservée, vasculaire et fibreuse, qui permet de bien la reconnaître.

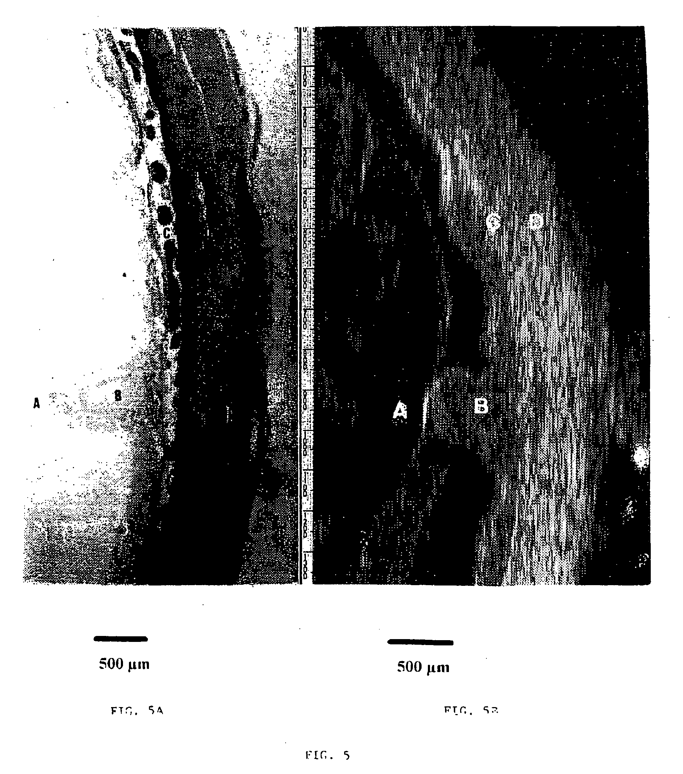

La Figure 5 montre un exemple de comparaison entre une coupe histologique macroscopique du Globe N° 3 (cliché de gauche), avec une coupe en microscopie ultrasonore (cliché de droite), passant dans la même région maculaire.

Sur le cliché histologique, il est possible de reconnaître un pli rétinien assez net (A) puis, vers le haut de l'image, une série de plis rétiniens moins proéminents. L'espace sous rétinien est occupé par un matériel nécrotique (B), puis la choroïde (C) est reconnaissable par sa structure vasculaire conservée. La paroi sclérale (D) d'aspect lamellaire ne présente pas d'anomalie.

Sur le cliché échographique, nous retrouvons le même pli rétinien (A) qui apparaît sous la forme d'un liseré échogène, suivant les mêmes ondulations que la coupe histologique. Sous la rétine, existe un petit espace moins échogène (B), correspondant à la nécrose sous rétinienne. Plus en arrière, existe une couche arciforme plus échogène (C), correspondant à la choroïde, puis la paroi sclérale apparaît moyennement échogène ( D ).

Les différents plis rétiniens reconnus par échographie correspondent donc à des plis rétiniens post mortem avec nécrose sous rétinienne mais avec aspect préservé de la choroïde.

Les coupes histologiques comparées aux B-scan de notre microscope ultrasonore, montrent qu'il est possible de reconnaître assez nettement la couche rétinienne proprement dite, mesurée en histologie à environ 400 microns, lorsque la rétine est séparée de la choroïde par le matériel nécrotique sous rétinien qui apparaît moins échogène.

Cette résolution, comparée à la résolution des sondes de 10 MHz, représente un progrès considérable apporté par la présente invention.

Cela permet d'analyser les épaississements maculaires, par exemple des DMLA, dont les plis rétiniens, retrouvés sur nos globes humains prélevés, représentent un bon modèle échographique.

La possibilité de mieux explorer cette région essentielle de l'oeil pour la bonne qualité de la perception visuelle, permet d'espérer une utilisation de plus en plus fréquente de cette technique, d'abord, comme simple moyen d'iconographier les différentes pathologies maculaires, puis comme moyen d'analyse quantitative des informations échographiques provenant de cette région oculaire à forte densité en photorécepteurs.

## Revendications

1. Utilisation d'un transducteur ultrasonore de fréquence nominale d'excitation supérieure à 20 MHz, de préférence comprise entre 50 et 80 MHz, **caractérisée en ce que** la focalisation est de l'ordre de 20 à 25 mm, pour la réalisation d'un appareil d'exploration échographique de tissus ou organes du corps humain ou animal, notamment du globe oculaire, en particulier du segment postérieur du globe oculaire, plus particulièrement de la région maculaire, ainsi que des tissus situés en arrière du globe oculaire comme les muscles oculomoteurs, la graisse orbitaire et le nerf optique.

2. Utilisation d'un transducteur ultrasonore de fréquence nominale d'excitation supérieure à 20 MHz, de préférence comprise entre 50 et 80 MHz, **caractérisée en ce que** la focalisation est de l'ordre de 20 à 25 mm, pour la mise en oeuvre d'un procédé d'exploration échographique de tissus ou organes du corps humain ou animal, notamment du globe oculaire, en particulier du segment postérieur du globe oculaire, plus particulièrement de la région maculaire, ainsi que des tissus situés en arrière du globe oculaire comme les muscles oculomoteurs, la graisse orbitaire et le nerf optique.

3. Utilisation selon la revendication 2, **caractérisée en ce que** le transducteur ultrasonore est décalé sur la pars plana pour éviter l'absorption du faisceau ultrasonore par le cristallin de l'oeil.

4. Utilisation selon l'une des revendications 2 ou 3, **caractérisée en ce que** le transducteur ultrasonore est protégé par une membrane en matière plastique.

5. Appareil d'exploration échographique comportant un système émetteur-récepteur haute fréquence, allant de 20 à 200 MHz, couplé à un transducteur ultrasonore **caractérisé en ce que** la focalisation dudit transducteur ultrasonore est de l'ordre de 20 à 25 mm, et **en ce que** ledit appareil comprend en outre un système d'amplification et de mémorisation du signal radiofréquence rétrodiffusé après l'exploration, de préférence associé à un système enregistreur du signal amplifié et/ou à un système de traitement du signal sous forme d'image, et/ou à un système de traitement du signal en vue de réaliser une caractérisation tissulaire.

6. Appareil selon la revendication 5, **caractérisé en ce que** le transducteur ultrasonore est agencé sous la forme d'une sonde pilotée de manière à se déplacer à proximité de la paroi antérieure oculaire.

7. Appareil selon la revendication 6, **caractérisé en ce que** le déplacement du transducteur ultrasonore est réalisé selon deux axes orthogonaux.

8. Appareil selon la revendication 6, **caractérisé en ce que** le déplacement du transducteur est un déplacement arciforme.

9. Appareil selon la revendication 7, **caractérisé en ce que** le transducteur ultrasonore est focalisé selon un troisième axe orthogonal aux deux axes orthogonaux de déplacement.

10. Appareil selon l'une des revendications 5 à 8, **caractérisé en ce que** le transducteur ultrasonore est focalisé sans déplacement à l'aide d'un système électronique de focalisation.

11. Appareil selon l'une des revendications 5 à 10, **caractérisé en ce que** le transducteur ultrasonore est protégé par une membrane en matière plastique.

## Claims

1. The use of an ultrasound transducer having a nominal excitation frequency greater than 20 MHz, preferably lying in the range 50 MHz to 80 MHz, the use being **characterized in that** the focal length is about 20 mm to 25 mm, for making a device for echographic exploration of tissues or organs of the human or animal body, specifically of the eyeball, in particular of the posterior segment of the eyeball, more particularly of the macular region, and also of tissues situated behind the eyeball such as the oculomotor muscles, eye socket fat, and the optic nerve.

2. The use of an ultrasound transducer having a nominal excitation frequency greater than 20 MHz, preferably lying in the range 50 MHz to 80 MHz, the use being **characterized in that** the focal length is about 20 mm to 25 mm, for implementing a method of echographic exploration of tissues or organs of the human or animal body, specifically of the eyeball, in particular of the posterior segment of the eyeball, more particularly of the macular region, and also of tissues situated behind the eyeball such as the oculomotor muscles, eye socket fat, and the optic nerve.

3. A use according to claim 2, **characterized in that** the ultrasound transducer is moved over the pars plana to avoid the ultrasound beam being absorbed by the lens of the eye.

4. A use according to claim 2 or 3, **characterized in that** the ultrasound transducer is protected by a membrane of plastics material.

5. A device for echographic exploration, the device comprising a transceiver system operating at high frequency, from 20 MHz to 200 MHz, that is coupled to an ultrasound transducer, the device being **characterized in that** the focal length of said ultrasound transducer is about 20 mm to 25 mm, and **in that** said device further comprises a system for amplifying and storing the radiofrequency signal as back-scattered after exploration, preferably associated with a system for recording the amplified signal and/or a system for processing the signal in the form of an image, and/or a system for processing the signal in order to perform tissue characterization.

6. A device according to claim 5, **characterized in that** the ultrasound transducer is implemented in the form of a probe controlled so as to move in the vicinity of the anterior wall of the eye.

7. A device according to claim 6, **characterized in that** the ultrasound transducer is displaced along two orthogonal axes.

8. A device according to claim 6, **characterized in that** the transducer is subjected to arcuate displacement.

9. A device according to claim 7, **characterized in that** the ultrasound transducer is focused along a third axis orthogonal to the two orthogonal displacement axes.

10. A device according to any one of claims 5 to 8, **characterized in that** the ultrasound transducer is focused without moving by using an electronic focusing system.

11. A device according to any one of claims 5 to 10, **characterized in that** the ultrasound transducer is protected by a membrane of plastics material.

## Patentansprüche

1. Verwendung eines Ultraschallwandlers mit nomineller Erregerfrequenz größer als 20 MHz, vorzugsweise zwischen 50 und 80 MHz, **dadurch gekennzeichnet, dass** die Fokussierung von der Größenordnung 20 bis 25 mm ist, zur Herstellung eines Geräts zur echographischen Untersuchung von Geweben oder Organen des menschlichen oder tierischen Körpers, besonders des Augapfels, insbesondere des hinteren Abschnitts des Augapfels, vor allem der Makularegion, sowie der hinter dem Augapfel gelegenen Gewebe, wie der okulomotorischen Muskeln, des Augenhöhlenfettgewebes und des Sehnervs.

2. Verwendung eines Ultraschallwandlers mit nomineller Erregerfrequenz größer als 20 MHz, vorzugsweise zwischen 50 und 80 MHz, **dadurch gekennzeichnet, dass** die Fokussierung von der Größenordnung 20 bis 25 mm ist, zur Durchführung eines Verfahrens zur echographischen Untersuchung von Geweben oder Organen des menschlichen oder tierischen Körpers, besonders des Augapfels, insbesondere des hinteren Abschnitts des Augapfels, vor allem der Makularegion, sowie der hinter dem Augapfel gelegenen Gewebe, wie der okulomotorischen Muskeln, des Augenhöhlenfettgewebes und des Sehnervs.

3. Verwendung gemäß Anspruch 2, **dadurch gekennzeichnet, dass** der Ultraschallwandler auf die Pars Plana verschoben wird, um die Absorption des Ultraschallstrahls durch die Linse des Auges zu vermeiden.

4. Verwendung gemäß einem der Ansprüche 2 oder 3, **dadurch gekennzeichnet, dass** der Ultraschallwandler von einer Kunststoffmembran geschützt wird.

5. Echographisches Untersuchungsgerät, umfassend ein Sender-Empfänger System mit hoher Frequenz, die von 20 bis 200 MHz reicht, gekoppelt an einen Ultraschallwandler, **dadurch gekennzeichnet, dass** die Fokussierung des Ultraschallwandlers von der Größenordnung 20 bis 25 mm ist, und dass das besagte Gerät außerdem ein System zur Verstärkung und Speicherung des nach der Untersuchung rückgestreuten Radiofrequenzsignals umfasst, das vorzugsweise mit einem System zur Erfassung des verstärkten Signals und/oder einem System zur Verarbeitung des Signals in Bildform und/oder mit einem System zur Verarbeitung des Signals im Hinblick auf die Herstellung einer Gewebecharakterisierung verbunden ist.

6. Gerät gemäß Anspruch 5, **dadurch gekennzeichnet, dass** der Ultraschallwandler in Form einer Sonde ausgebildet ist, die so geführt wird, dass sie sich in der Nähe der vorderen Augenwand bewegt.

7. Gerät gemäß Anspruch 6, **dadurch gekennzeichnet, dass** die Bewegung des Ultraschallwandlers entlang zweier senkrechter Achsen erfolgt.

8. Gerät gemäß Anspruch 6, dass die Bewegung des Wandlers eine bogenförmige Bewegung ist.

9. Gerät gemäß Anspruch 7, **dadurch gekennzeichnet, dass** der Ultraschallwandler entlang einer dritten Achse fokussiert wird, die senkrecht zu den beiden senkrechten Bewegungsachsen ist.

10. Gerät gemäß einem der Ansprüche 5 bis 8, **dadurch gekennzeichnet, dass** der Ultraschallwandler ohne Bewegung mit Hilfe eines elektronischen Fokussierungssystems fokussiert wird.

11. Gerät gemäß einem der Ansprüche 5 bis 10, **dadurch gekennzeichnet, dass** der Ultraschallwandler von einer Kunststoffmembran geschützt ist.
